# EUROPÄISCHE PATENTSCHRIFT

(11) **EP 0 691 338 B1**
(45) Veröffentlichungstag und Bekanntmachung des Hinweises auf die Patenterteilung: **03.09.1997**
(21) Anmeldenummer: 95109782.3
(22) Anmeldetag: 23.06.1995
(51) Int. Cl.: C07D 323/06

(54) **Verfahren zur Herstellung von Trioxan**
Process for the preparation of trioxane
Procédé de préparation de trioxanne

(30) Priorität: 06.07.1994 DE 4423618
(43) Veröffentlichungstag der Anmeldung: 10.01.1996
(73) Patentinhaber: HOECHST AKTIENGESELLSCHAFT, 65929 Frankfurt am Main (DE)
(72) Erfinder: Hoffmockel, Michael, Dr., D-65527 Niedernhausen (DE); Sextro, Günter, Dr., D-65207 Wiesbaden (DE); Emig, Gerhard, Prof. Dr., D-91058 Erlangen (DE); Kern, Frank, Dr., D-76870 Kandel (DE)

(56) Entgegenhaltungen:
- DE-A- 3 106 476

## Beschreibung

Die Erfindung bezieht sich auf ein Verfahren zur kontinuierlichen Herstellung von Trioxan in der Gasphase durch heterogene Katalyse mit Wolframomolybdophosphorsäuren der Zusammensetzung H₃PWₙMoₘO₄₀* x H₂O (n = 4 bis 8, m = 12 - n, x = 0 bis 32) als Katalysator.

Trioxan kann aus wäßrigen Formaldehydlösungen mit sauren Katalysatoren hergestellt werden. Kennzeichnend für diese Prozesse ist der hohe Energieverbrauch beim Verdampfen von Wasser, das durch die Eduktströme in den Prozeß eingetragen wird.

Es gibt verschiedene Vorschläge, Trioxan durch eine Gasphasentrimerisierung von Formaldehyd herzustellen, jedoch wird in vielen Fällen mit Formaldehydströmen unterschiedlichen Wassergehaltes gearbeitet. Beim Arbeiten mit wasserhaltigem Formaldehyd treten durch Ablagerungen von Polyoxymethylen auf der Katalysatoroberfläche Probleme auf.

Ein Verfahren zur Herstellung von Trioxan mittels eines sauren Harzionenaustauschers ist bekannt (DE-C-1 593 990). Ebenfalls bekannt ist ein Katalysator für die Gasphasentrimerisierung in Form von Phosphorsäure oder Schwefelsäure auf SiO₂-Trägern (AT-B 252 913). Außerdem sind verschiedene Heteropolysäuren als Katalysatoren bei der Gasphasentrimerisierung von Formaldehyd eingesetzt worden (JP-A 59-25387). Bei diesen Verfahren sind die Ausbeuten nicht zufriedenstellend.

Fortschritte wurden erzielt durch den Einsatz sogenannten wasserfreien Formaldehyds, das heißt Formaldehyd mit Wassergehalten ≤ 1%. Dadurch konnten sowohl die Ablagerungen von Polyoxymethylen auf der Katalysatoroberfläche vermindert bzw. verhindert werden als auch die Ausbeuten gesteigert werden. Bekannt sind Trimerisierungskatalysatoren in Form von Vanadylhydrogenphosphat (EP 604884) und der Heteropolysäure 1-Vanado-11-Molybdophosphorsäure (H₄PVMo₁₁O₄₀)(1-V-Säure) (EP 606056). Diese Katalysatoren sind aber noch nicht zufriedenstellend hinsichtlich eines hohen Umsatzes und gleichzeitig hoher Selektivität.

Es bestand die Aufgabe diesen Nachteil zu beseitigen.

Gelöst wurde die Aufgabe durch ein Verfahren, welches sich von den bekannten Verfahren durch den Katalysator unterscheidet, der zu einer hohen Selektivität und gleichzeitig einem Umsatz führt, der nahe des thermodynamisch möglichen Umsatzes liegt.

Gegenstand der Erfindung ist ein Verfahren zur Herstellung von Trioxan aus Formaldehyd in der Gasphase in Gegenwart eines Katalysators der allgemeinen Formel H₃PWₙMoₘO₄₀* x H₂O (n = 4 bis 8, m = 12 - n, x = 0 bis 32) (im folgenden MoW-Säure genannt).

Diese als Katalysatoren dienenden Heteropolysäuren können nach der Methode von WU (J. Biol. Chem. 43 (1920) 189) hergestellt werden.

Der Katalysator kann in Substanz oder mit Presshilfen eingesetzt werden.Trägerlose Katalysatoren können durch Vermischen von gesiebter MoW-Säure, Korngröße z.B. 150 µm, mit der gewünschten Menge an Presshilfe und anschließendes Verpressen zu Pellets der gewünschten Größe hergestellt werden. Als Presshilfen werden Stoffe verwendet, die sich bei der Reaktion inert verhalten, z.B. feinverteilte Kieselsäure (SiO₂), Aluminiumoxid oder -hydroxid. Vorteilhaft ist die Verwendung des Katalysators auf einem Trägermaterial. Als Trägermaterial können inerte Materialien, z.B. Pellets aus Siliciumcarbid, Siliciumdioxid oder Aluminiumoxid, eingesetzt werden.

Der für dieses Verfahren verwendete Formaldehyd kann einen geringen Wassergehalt aufweisen, d.h. bis zu 1 Gew.-% Wasser enthalten. Bevorzugt ist Formaldehyd mit einem Restwassergehalt von bis zu 50 ppm, den man im allgemeinen als wasserfreien Formaldehyd bezeichnet. Enthält der Gasstrom kein Wasser, so liegt der Katalysator unter Reaktionsbedingungen in wasserfreier Form oder als Hexahydrat vor.

Der Temperaturbereich für die Reaktion liegt gewöhnlich bei 80 bis 150°C. Bevorzugt ist der Bereich von 100 bis 120°C.

Die Kontaktdauer, bei der wenig Nebenprodukte (< 4 %) auftreten, liegt bei 1 bis 30 Sekunden, vorzugsweise 1 bis 10 Sekunden. Längere Kontaktzeiten sind möglich, jedoch besteht dann die Gefahr vermehrter Nebenproduktbildung.

Die Reaktion wird durch den Partialdruck von Formaldehyd beeinflußt. Der Katalysator hat über einen großen Druckbereich eine hohe Selektivität für die Bildung von Trioxan. Der Eingangspartialdruck von Formaldehyd beträgt im allgemeinen 0,5 bis 5 bar, vorzugsweise 0,5 bis 2 bar.

Die Katalysatoren zur Herstellung von Trioxan wurden in einer Apparatur, wie sie in Figur 1 schematisch gezeigt wird, untersucht. Die Apparatur besteht aus drei Teilen:
- Dosierung (1)
- Bildungsreaktor (2)
- Analytik und Auswertung (3)

Zur Untersuchung wird Formaldehyd (FA) in die Apparatur eingeschleust und- wenn gewünscht - mit einem Trägergas (TG) vermischt. Als Trägergase können Wasserstoff, Edelgase wie Helium, Argon, Krypton oder Xenon dienen, bevorzugt wird Stickstoff eingesetzt.

Mit Hilfe eines thermischen Massendurchflußreglers (M) kann das Trägergas dem Formaldehyd-Eingangsstrom zugefügt werden. Durch Einsatz variabler Mengen an Trägergas zum Verdünnen des Formaldehydeingangsstromes können in einfacher Weise die gewünschten Partialdrücke an Formaldehyd eingestellt werden.

Der Formaldehyd-Gasstrom wurde in den Reaktor (R) geführt, der den Katalysator enthält. Die Temperatur im Reaktor wurde über einen Thermostaten geregelt und an drei verschiedenen Punkten entlang der Katalysatorschüttung gemessen (radial). Als Wärmeträger wurde Siliconöl benutzt. Andere Wärmeträger wie Mineralöle sind ebenfalls einsetzbar. Die Temperaturen wurden aufgezeichnet und geben Auskunft über den stabilen Betriebszustand des Reaktors während eines Versuchs.

Der aus dem Reaktor austretende Ausgangsstrom mit den entstandenen Reaktionsprodukten wurde durch Absorption (A) mit Wasser aufgefangen. Das gebildete Trioxan kann daraus in bekannter Weise durch Extraktion gewonnen werden.

Zur Bestimmung des Umsatzes wurde die Gaszusammensetzung der Eingangs- und -ausgangsströme des Reaktors online bestimmt. Dabei wurden während des Betriebs über pneumatische Kugelhähne (PV) automatisch Proben gezogen und in einem Gaschromatographen (GC) analysiert. Die Probenahme erfolgte in regelmäßigen Abständen, z.B. alle zehn Minuten, und wurde über einen Computer gesteuert.

Der Gleichgewichtsumsatz (U_{gl}) wird aus den gegebenen Partialdrücken und den Gleichgewichtskonstanten nach Busfield und Merigold ("The Gas-Phase Equilibrium between Trioxane and Formaldehyde", J. Am. Chem. Soc. (A), 1969, S. 2975) berechnet. Bezieht man den experimentell gefundenden Umsatz Uₑₓₚ auf den Gleichgewichtsumsatz, erhält man den relativen Umsatz Uᵣₑₗ.

Relativer Umsatz Uᵣₑₗ = 100 * Uₑₓₚ / U_{gl} .

Die Anordnung der Apparatur für das Verfahren gemäß der Erfindung sowie die Dimensionen können den jeweiligen Bedingungen angepaßt werden.

Besonders hervorzuheben sind bei dem Verfahren gemäß der Erfindung folgende Vorteile
1. Erreichen von hohen Raumzeitausbeuten (RZA) [kg/m³∗h],
2. keine Nebenprodukte,
3. moderate Wärmeentwicklung im Synthesereaktor.

### Beispiele:

In allen aufgeführten Beispielen wurde Stickstoff als Trägergas und Referenzgas für die Analysen benutzt. Der Wassergehalt des Formaldehydeingangsstromes lag unter 50 ppm.

Die MoW-Säuren, die als Katalysator dienen, wurden nach der Methode von WU (loc. cit.) hergestellt.

Zur Herstellung der Tränkkatalysatoren wird die MoW-Säure, in Wasser gelöst, zum Tränken oder Imprägnieren der Träger verwendet. Dazu werden die Katalysatorträger mit der Lösung überschichtet, die in den Poren der Träger enthaltene Luft durch Verminderung des Drucks im Imprägniergefäß abgesaugt, die überstehende Lösung abgegossen und die imprägnierten Träger bei 373 K an der Luft getrocknet. Sollen die Träger nur getränkt werden, kann die Evakuierung entfallen.

In den folgenden Beispielen wurden auf SiC-Träger imprägnierte MoW-Säuren verwendet.

Der Bildungsreaktor bestand aus einem Edelstahlrohrreaktor von 150 mm Länge und einem Innendurchmesser von 16 mm.

Die Ergebnisse der folgenden Versuche sind in Tabelle 1 zusammengestellt.
1) 0,56 g des Katalysators H₃PW₄Mo₈O₄₀ auf 18,12 g SiC-Pellets (Durchmesser 3 mm, Höhe 3 mm) wurden im Reaktor eingesetzt. Der Eingangspartialdruck von Formaldehyd betrug 790 mbar. Das Volumen der Katalysatorschüttung betrug 20 cm³. Die Kontaktzeit betrug 6,2 Sekunden.
2) 0,62 g des Katalysators H₃PW₆Mo₆O₄₀ auf 18,14 g SiC-Pellets (Durchmesser 3 mm, Höhe 3 mm) wurden im Reaktor eingesetzt. Der Eingangspartialdruck von Formaldehyd betrug 780 mbar. Das Volumen der Katalysatorschüttung betrug 20 cm³. Die Kontaktzeit betrug 6,5 Sekunden.
3) 0,53 g des Katalysators H₃PW₈Mo₄O₄₀ auf 18,02 g SiC-Pellets (Durchmesser 3 mm, Höhe 3 mm) wurden im Reaktor eingesetzt. Der Eingangspartialdruck von Formaldehyd betrug 802 mbar. Das Volumen der Katalysatorschüttung betrug 20 cm³. Die Kontaktzeit betrug 6,0 Sekunden.
Es folgen Vergleichsbeispiele.
4) 0,645 g des Katalysators H₃PW₂Mo₁₀O₄₀ auf 18,15 g SiC-Pellets (Durchmesser 3 mm, Höhe 3 mm) wurden im Reaktor eingesetzt. Der Eingangspartialdruck von Formaldehyd betrug 818 mbar. Das Volumen der Katalysatorschüttung betrug 20 cm³. Die Kontaktzeit betrug 6,25 Sekunden.
5) 0,535 g des Katalysators H₃PW₁₀Mo₂O₄₀ auf 18,27 g SiC-Pellets (Durchmesser 3 mm, Höhe 3 mm) wurden im Reaktor eingesetzt. Als Nebenprodukte entstanden Ameisensäuremethylester und geringe Megen Orthoameisensäuretrimethylester. Der Eingangspartialdruck von Formaldehyd betrug 780 mbar. Das Volumen der Katalysatorschüttung betrug 20 cm³. Die Kontaktzeit betrug 6,4 Sekunden.
6) 0,563 g des Katalysators 1-V-Säure auf 16,7 g zylindrischer SiC-Pellets (Durchmesser 6 mm, Höhe 6 mm) wurden im Reaktor eingesetzt. Der Eingangspartialdruck von Formaldehyd betrug 1150 mbar, das Volumen der Katalysatorschüttung betrug 16,6 cm³. Die Verweilzeit betrug 5,1 Sekunden.
7) 0,9 g des Katalysators 1-V-Säure auf 26,65 g zylindrischer SiC-Pellets (Durchmesser 6 mm, Höhe 6 mm) wurden im Reaktor eingesetzt. Der Eingangspartialdruck von Formaldehyd betrug 950 mbar, das Volumen der Katalysatorschüttung betrug 26,7 cm³. Die Verweilzeit betrug 5,1 Sekunden.

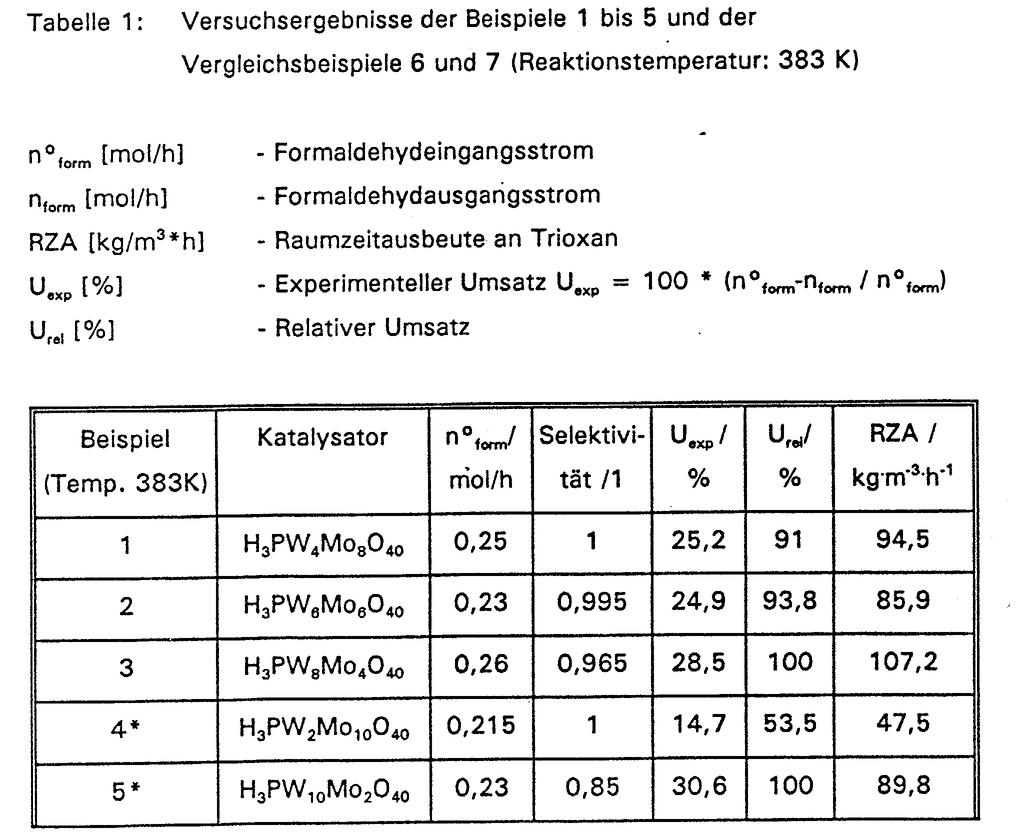

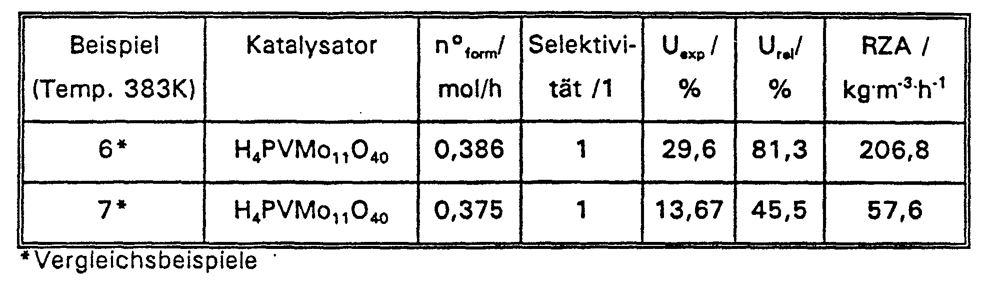

Die Katalysatoren gemäß der Erfindung zeigen bei einer Versuchsdauer von 300 Stunden keine Desaktivierung.

Wie die Beispiele 1 bis 3 zeigen, besitzen die MoW-Säuren gemäß der Erfindung sowohl hohe Selektivitäten für die Bildung von Trioxan als auch hohe Umsätze nahe dem Gleichgewichtsumsatz, verbunden mit hohen Raumzeitausbeuten.

In den Vergleichsbeispielen 4 und 5 werden MoW-Säuren eingesetzt, die eine andere Zusammensetzung haben als die MoW-Säuren gemäß der Erfindung. Hier wird entweder eine hohe Selektivität bei einem niedrigen relativen Umsatz (Beispiel 4) oder ein hoher Umsatz bei einer niedrigen Selektivität (Beispiel 5) erreicht. Diese Beispiele verdeutlichen, daß nicht jede MoW-Säure als Katalysator zu einem hohen Umsatz bei einer hohen Selektivität führt.

Vergleichsbeispiele 6 und 7 dienen zum Vergleich mit einem anderen Katalysatorsystem, der 1-Vanado-11-Molybdophosphorsäure. Das Katalysatorsystem liefert bei hohen Selektivitäten nur mäßige relative Umsätze.

Im Vergleichsbeispiel 7, welches mit 950 mbar Formaldehyd-Eingangspartialdruck noch am besten mit den Beispielen 1 bis 3 (ca. 800 mbar] vergleichbar ist, liegen sowohl der relative Umsatz als auch die Raumzeitausbeute deutlich niedriger. Durch zusätzliche Steigerung des Eingangspartialdrucks auf 1150 mbar konnten Verbesserungen erreicht werden (Beispiel 6). Höhere Drücke sind aber immer mit einem größeren Risiko der Bildung von Ablagerungen von Polyoxymethylen verbunden.

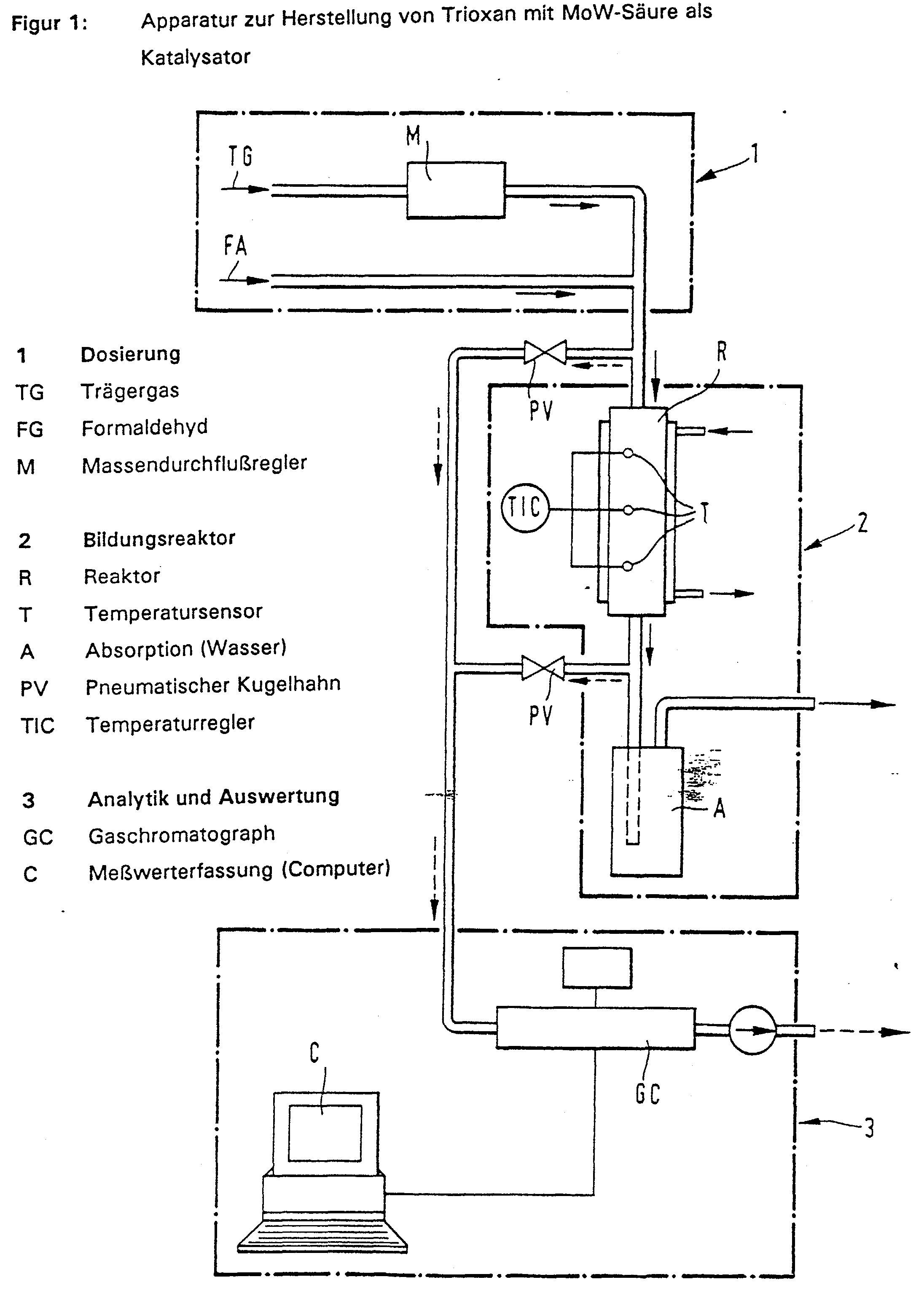

## Patentansprüche

1. Verfahren zur Herstellung von Trioxan aus Formaldehyd in der Gasphase, dadurch gekennzeichnet, daß als Katalysator Wolframo-Molybdophosphorsäuren der Zusammensetzung H₃PWₙMoₘO₄₀* x H₂O, wobei n = 4 bis 8, m = 4 bis 8, n + m = 12, x = 0 bis 32 ist, verwendet werden.

2. Verfahren nach Anspruch 1, dadurch gekennzeichnet, daß der Katalysator auf inerte Trägermaterialien aufgebracht wird.

3. Verfahren nach Anspruch 1 oder 2, dadurch gekennzeichnet, daß als Trägermaterial Siliciumcarbid verwendet wird.

4. Verfahren nach einem oder mehreren der Ansprüche 1 bis 3, dadurch gekennzeichnet, daß Formaldehyd mit einem geringen Wassergehalt von weniger als 1 Gew.-%, insbesondere mit einem Wassergehalt von weniger als 50 ppm, eingesetzt wird.

5. Verfahren nach einem oder mehreren der Ansprüche 1 bis 4, dadurch gekennzeichnet, daß die Reaktion bei 80 bis 150°C, vorzugsweise bei 100 bis 120°C durchgeführt wird.

6. Verfahren nach einem oder mehreren der Ansprüche 1 bis 5, dadurch gekennzeichnet, daß die Kontaktdauer 1 bis 30 Sekunden, vorzugsweise 1 bis 10 Sekunden beträgt.

7. Verfahren nach einem oder mehreren der Ansprüche 1 bis 6, dadurch gekennzeichnet, daß der Eingangspartialdruck des Formaldehyds 0,5 bis 5 bar, vorzugsweise 0,5 bis 2 bar beträgt.

8. Verfahren nach einem oder mehreren der Ansprüche 1 bis 7, dadurch gekennzeichnet, daß die Reaktion in Anwesenheit oder Abwesenheit eines Trägergases durchgeführt wird.

9. Verfahren nach einem oder mehreren der Ansprüche 1 bis 8, dadurch gekennzeichnet, daß Stickstoff als Trägergas verwendet wird.

## Claims

1. A process for preparing trioxane from formaldehyde in the gas phase, wherein tungstomolybdophosphoric acids of the composition H₃PWₙMoₘ0₄₀·xH₂O where n = 4 to 8, m = 4 to 8, n + m = 12, x = 0 to 32 are used as catalyst.

2. The process as claimed in claim 1, wherein the catalyst is applied to inert support materials.

3. The process as claimed in claim 1 or 2, wherein silicon carbide is used as support material.

4. The process as claimed in one or more of claims 1 to 3, wherein formaldehyde having a low water content of less than 1% by weight, particularly having a water content of less than 50 ppm, is used.

5. The process as claimed in one or more of claims 1 to 4, wherein the reaction is carried out at from 80 to 150°C, preferably at from 100 to 120°C.

6. The process as claimed in one or more of claims 1 to 5, wherein the contact time is from 1 to 30 seconds, preferably from 1 to 10 seconds.

7. The process as claimed in one or more of claims 1 to 6, wherein the inlet partial pressure of the formaldehyde is from 0.5 to 5 bar, preferably from 0.5 to 2 bar.

8. The process as claimed in one or more of claims 1 to 7, wherein the reaction is carried out in the presence or absence of a carrier gas.

9. The process as claimed in one or more of claims 1 to 8, wherein nitrogen is used as carrier gas.

## Revendications

1. Procédé de préparation de trioxanne à partir de formaldéhyde en phase gazeuse, caractérisé en ce qu'on utilise en tant que catalyseur des acides tungstomolybdophosphoriques ayant la composition H₃PWₙMoₘO₄₀*xH₂O, où n = 4 à 8, m = 4 à 8, n+m = 12, x = 0 à 32.

2. Procédé selon la revendication 1, caractérisé en ce que le catalyseur est appliqué sur des matériaux supports inertes.

3. Procédé selon la revendication 1 ou 2, caractérisé en ce qu'on utilise comme matériau support du carbure de silicium.

4. Procédé selon l'une ou plusieurs des revendications 1 à 3, caractérisé en ce que le formaldéhyde est utilisé avec une faible teneur en eau, inférieure à 1 % en poids, en particulier avec une teneur en eau inférieure à 50 ppm.

5. Procédé selon l'une ou plusieurs des revendications 1 à 4, caractérisé en ce que la réaction est mise en oeuvre à une température de 80 à 150°C, de préférence de 100 à 120°C.

6. Procédé selon l'une ou plusieurs des revendications 1 à 5, caractérisé en ce que la durée du contact est de 1 à 30 secondes, de préférence de 1 à 10 secondes.

7. Procédé selon l'une ou plusieurs des revendications 1 à 6, caractérisé en ce que la pression partielle d'entrée du formaldéhyde est de 0,5 à 5 bar, de préférence de 0,5 à 2 bar.

8. Procédé selon l'une ou plusieurs des revendications 1 à 7, caractérisé en ce que la réaction est mise en oeuvre en présence ou en l'absence d'un gaz support.

9. Procédé selon l'une ou plusieurs des revendications 1 à 8, caractérisé en ce qu'on utilise de l'azote en tant que gaz support.
